# FASCICULE DE BREVET EUROPEEN

(11) **EP 2 488 049 B1**
(45) Date de publication et mention de la délivrance du brevet: **26.08.2015**
(21) Numéro de dépôt: 10751993.6
(22) Date de dépôt: 12.07.2010
(51) Int. Cl.: A23K 1/14, A23K 1/18, A23L 1/30, A61K 35/74, A61K 36/324, A61K 36/76, A61K 36/896, A61K 36/9066

(54) **COMPLÉMENT ALIMENTAIRE DE SANTÉ ANIMALE OU HUMAINE POUR LE SOULAGEMENT ET LE SOUTIEN DES ARTICULATIONS**
NAHRUNGSERGÄNZUNG FÜR DIE GESUNDHEIT VON TIEREN ODER MENSCHEN ZUR ENTLASTUNG UND UNTERSTÜTZUNG VON GELENKEN
ANIMAL OR HUMAN HEALTH FOOD SUPPLEMENT FOR RELIEVING AND SUPPORTING JOINTS

(30) Priorité: 09.09.2009 FR 0956130
(43) Date de publication de la demande: 22.08.2012
(73) Titulaire: SOLUVEG SA, 2341 Luxembourg (LU)
(72) Inventeur: DHIMOLEA, Michel, F-76130 Mont Saint Aignan (FR); FRESU Antonello, 6914 Roodt sur Syre (LU)
(74) Mandataire: Office Freylinger
(86) Numéro de dépôt international: PCT/FR2010/051460
(87) Numéro de publication internationale: WO 2011/030021

(56) Documents cités:
- KR-A- 20040 057 103
- US-A- 5 494 668
- US-A- 5 916 565
- US-A1- 2006 040 000
- US-B1- 6 224 871
- KHANNA ET AL: "Natural products as a gold mine for arthritis treatment" CURRENT OPINION IN PHARMACOLOGY, ELSEVIER SCIENCE PUBLISHERS, NL LNKD- DOI:10.1016/J.COPH.2007.03.002, vol. 7, no. 3, 1 juin 2007 (2007-06-01), pages 344-351, XP022078335 ISSN: 1471-4892
- NAGUIB Y: "THE JOINT HEALTH MARKET: EXAMINING INGREDIENTS FOR JOINT CONDITIONS" NUTRACEUTICALS WORLD, RODMAN PUBLICATIONS, INC., RAMSEY, NJ, US, vol. 6, no. 4, 1 avril 2003 (2003-04-01), pages 48-56, XP009064202 ISSN: 1531-0671
- TORFS S. ET AL.: 'Anti-inflammatory phytotherapeutics: a valuable alternative to NSAID treatment in horses ?' VLAAMS DIERGENEESKUNDIG TIJDSCHRIFT vol. 78, 2008, page 161-170

## Description

L'invention concerne un complément alimentaire de santé animale ou humaine.

L'invention concerne plus un complément alimentaire de santé animale ou humaine à base aqueuse comportant des principes actifs d'origine végétale.

On connaît de nombreux exemples de compléments alimentaires de ce type.

De tels compléments alimentaires sont par exemple dans le domaine de la santé animale dans le milieu hippique afin d'améliorer les performances des chevaux.

US 6 224 871 B1 décrit un complément alimentaire pour la santé des articulations comportant des extraits de Boswellia serrata, Curcuma longa et Yucca schigidera.

US 5 494 668 A décrit une composition permettant de traiter les maladies dégénératives des muscles et du squelette comme l'arthrite comportant des extraits de Boswellia serrata et de Curcuma longa.

US 2006/040000 A1 divulgue un supplément diététique pour le traitement de l'arthrite comportant entre autres des extraits de Curcuma, Boswellia et Salix Alba.

KHANNA et al: "Natural products as a gold mine for arthritis treatment" Current Opinion In Pharmacology, Elsevier Science Publishers, vol. 7, no. 3, 1 juin 2007, pages 344-351, liste des produits bénéfiques à la santé des articulations et cite Boswellia serrata et Curcuma longa.

US 5 916 565 A divulgue une composition pour le traitement des maladies articulaires comportant des extraits de Curcuma longa (« turmeric ») et Yucca.

NAGUIB Y: "The joint health market: examining ingrédients for joint conditions", Nutraceuticals World, Rodman Publications, Inc., Ramsey, NJ, US, vol. 6, no.4, 1 avril 2003, pages 48-56, liste des produits bénéfiques à la santé des articulations et cite Boswellia serrata et Curcuma longa et Salix.

KR 2004 0057103 A divulgue une composition pour le traitement des maladies dégénératives des articulations comportant des algues qui contiennent au moins 10% d'un complexe de polyphloroglucinol. Les algues comportent entre autres les algues de nom binominal Porphyra tenera, Costaria costata, Hizikia fusiforme, Undaria pinnatifida, Meristotheca papulosa, Enteromorpha, Porphyra tenera, Laminaria japonica. Rheum coreanum Nakai., Acanthogobius hasta, Gigartina tenella, Gallicrex cinerea cineria, G. furcata, Campylaephora hypnaeoides. Spirulina, Gelidium amansii, Chondrus ocellatus Holmes, Codium Fragile, Chlorella.

Toutefois, de tels compléments doivent impérativement être exempts de substances telles que notamment la Caféine, la Thébromine, La Théophylline, Atropine, Scopolamine, Morphine, Methylbufoténine, Diméthyltryptamine et Bufoténine qui sont considérées comme substances dopantes.

Le complément objet de l'invention est exclusivement constitué de composants issus de végétaux ou d'algues ne procurant pas d'effet dopant.

Le complément objet de l'invention permet une diminution de la fatigue des articulations des chevaux, leur permettant ainsi de soutenir un effort important pendant une durée accrue.

Le même complément peut être appliqué à d'autres espèces animales dont l'Homme.

Dans ce but, l'invention propose un complément alimentaire du type décrit précédemment, caractérisé en ce que lesdits principes actifs comportent au moins:
- des extraits de Curcumine, de variété botanique Curcuma Longa,
- des extraits d'écorce de Saule Blanc, de variété Botanique Salix Alba,
- des extraits de racine de Yucca, de variété Botanique Yucca Schidigera,
- des extraits de Boswellia, de variété botanique Boswellia Serrata,
pour permettre notamment le soulagement et le soutien des articulations sensibles et/ou vieillissantes.

Selon d'autres caractéristiques de l'invention :
- le complément alimentaire comporte de surcroît des extraits de Spiruline, de variété Spirulina Arthrospira Platensis,
- le complément alimentaire est constitué d'un mélange dont les pourcentages massiques comportent 60,4 % d'eau et au moins :
   - 2,6% d'extraits de Curcumine,
   - 1,8% d'extraits d'écorce de Saule Blanc,
   - 1,8% d'extraits de racine de Yucca,
   - 0.7% d'extraits de Boswellia,
   - 2% d'extraits de Spiruline,
   chacun de ces dosages étant réalisé avec une marge d'erreur de 0.1% en excès ou en défaut.
- le complément alimentaire comporte des principes inactifs, notamment des agents de goût et/ou acidifiants qui comportent au moins :
   - de l'acide citrique,
   - des arômes de fruit,
   - du Sorbate de potassium,
   - de la Gomme de Xanthane,
   - du Saccharose.
- l'arôme de fruit est un arôme de pomme verte.
- le mélange comporte de surcroît des pourcentages massiques de :
   - 2,3% d'acide citrique,
   - 2% d'arôme de pomme verte,
   - 0,3% de Sorbate de potassium,
   - 1 % de Gomme de Xanthane,
   - 25,1 % de Saccharose,
   chacun de ces dosages étant réalisé avec une marge d'erreur de 0.1% en excès ou en défaut.

L'invention concerne aussi un procédé de fabrication d'un complément alimentaire selon l'invention, caractérisé en ce qu'il comporte successivement :
- une première étape de préparation de la base aqueuse au cours de laquelle on chauffe l'eau à 40°C et au cours de laquelle on la soumet à l'action d'un mélangeur rotatif tournant à 500 tours/minute,
- une deuxième étape d'incorporation au cours de laquelle on incorpore les arômes de pomme verte et le Saccharose et au cours de laquelle on soumet le mélange à l'action du mélangeur rotatif tournant à 1500 tours/minute,
- une troisième étape au cours de laquelle on incorpore les extraits de Curcumine, les extraits d'écorce de Saule Blanc, les extraits de racine de Yucca, les extraits de Boswellia, l'acide citrique et le Sorbate de potassium et au cours de laquelle on soumet le mélange à l'action du mélangeur rotatif tournant à 500 tours/minute,
- une quatrième étape au cours de laquelle on incorpore la gomme de Xanthane et au cours de laquelle on soumet le mélange à l'action du mélangeur rotatif tournant à 1500 tours/minute,
- une cinquième étape de refroidissement au cours de laquelle on laisse refroidir le mélange jusqu'à ce que sa température atteigne la température ambiante.

Selon d'autres caractéristiques du procédé :
- l'ensemble des quatre premières étapes s'étend sur une durée comprise entre 20 et 30 minutes,
- au cours des quatre premières étapes, on utilise un mélangeur rotatif pourvu d'une hélice quadripale.

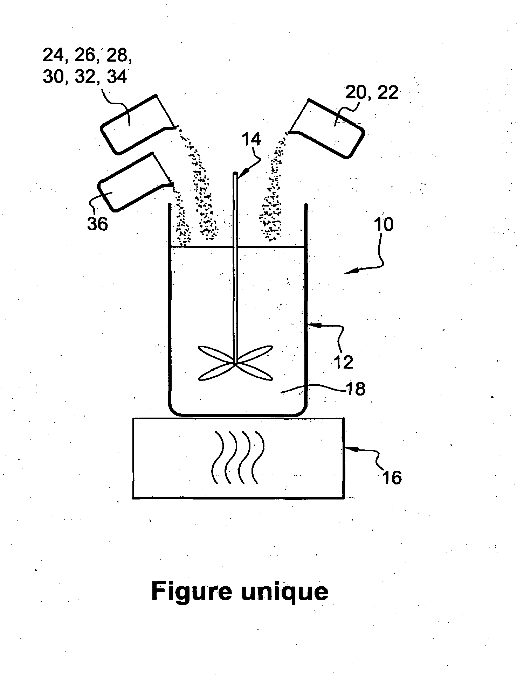

D'autres caractéristiques et avantages de l'invention apparaîtront à la lecture de la description détaillée qui suit pour la compréhension de laquelle on se reportera aux dessins annexés dans lesquels la figure unique est une vue schématique d'un dispositif permettant de réaliser complément alimentaire objet de l'invention.

On a représenté sur la figure unique un mélangeur 10 destiné à permettre la fabrication du complément alimentaire objet de l'invention.

De manière connue le mélangeur 10 comporte une cuve 12 dans laquelle plonge un moyen d'agitation 14, notamment un mélangeur rotatif constitué d'une hélice quadripale 14. La cuve 12 repose sur un moyen de chauffage constitué d'une plaque chauffante 16 qui permet d'élever la température de son contenu.

Cette configuration n'est évidemment pas limitative de l'invention. Le moyen d'agitation pourrait être constitué d'un agitateur magnétique, et le moyen de chauffage pourrait être constitué d'un thermoplongeur.

Conformément à l'invention, le mélangeur 10 permet de mettre en oeuvre un procédé de fabrication d'un complément 18 alimentaire à base aqueuse selon l'invention.

De manière connue, ce complément 18 à base aqueuse comporte des principes actifs d'origine végétale.

Conformément à l'invention, ces principes actifs comportent au moins :
- des extraits de Curcumine, de variété botanique Curcuma Longa,
- des extraits d'écorce de Saule Blanc, de variété Botanique Salix Alba,
- des extraits de racine de Yucca, de variété Botanique Yucca Schidigera, et
- des extraits de Boswellia, de variété botanique Boswellia Serrata,
pour permettre notamment le soulagement et le soutien des articulations sensibles et/ou vieillissantes.

L'incorporation d'au moins chacun de ces composant au cours de tests réalisés dans le milieu hippique a permis de mettre en évidence un assouplissement des articulations des chevaux les ayant ingéré, avec pour résultat une meilleure résistance à un effort soutenu.

Avantageusement, le complément 18 comporte de surcroît des extraits de Spiruline, de variété Spirulina Arthrospira Platensis.

La spiruline est une micro-algue de la famille des cyanobactéries. Sa digestibilité est excellente, et elle contient de 50 à 70% de protéines, du fer, et des vitamines A et B12 en grandes quantités.

Dans le mode de réalisation préféré de l'invention, le complément 18 est constitué d'un mélange dont les pourcentages massiques comportent 60,4% d'eau et au moins :
- 2,6% d'extraits de Curcumine,
- 1,8% d'extraits d'écorce de Saule Blanc,
- 1,8% d'extraits de racine de Yucca,
- 0.7% d'extraits de Boswellia,
- 2% d'extraits de Spiruline.

Chacun de ces dosages est réalisé avec une marge d'erreur de 0.1% en excès ou en défaut.

De préférence, les extraits de Curcumine comportent une teneur de 93 à 97% de Curcumine, et les extraits d'écorce de Saule Blanc sont constitués de plus de 92% d'extrait sec d'écorce de Saule Blanc.

Pour permettre une absorption aisée du complément 18 par un cheval, ce complément 18 comporte de surcroît des principes inactifs, notamment des agents de goût et/ou acidifiants.

Ces principes comportent au moins de l'acide citrique, des arômes de fruit, du Sorbate de potassium, de la Gomme de Xanthane, et du Saccharose.

L'arôme de fruit est un arôme de pomme verte, mais cette configuration n'est pas limitative de l'invention.

Dans cette configuration, les principes inactifs sont eux aussi dosés de manière précise, avec une marge d'erreur de 0.1 % en excès ou en défaut.

Ainsi, le mélange comporte des pourcentages massiques des principes actifs de 2,3% d'acide citrique, 2% d'arôme de pomme verte, 0,3% de Sorbate de potassium, 1% de Gomme de Xanthane, et 25,1% de Saccharose.

Le procédé de fabrication du complément alimentaire équin 18 consiste donc à réliser sous certaines conditions le mélange des extraits de Curcumine, des extraits d'écorce de Saule Blanc, des extraits de racine de Yucca, des extraits de Boswellia, de l'acide citrique, des arômes de pomme verte, du Sorbate de potassium, de la Gomme de Xanthane et du Saccharose.

A cet effet, le procédé comporte successivement :
- une première étape de préparation "ET1" de la base aqueuse au cours de laquelle on chauffe l'eau à 40°C et au cours de laquelle on la soumet à l'action du mélangeur rotatif 14 tournant à 500 tours/minute,
- une deuxième étape d'incorporation "ET2" au cours de laquelle on incorpore les arômes 20 de pomme verte et le Saccharose 22 et au cours de laquelle on soumet le mélange à l'action du mélangeur rotatif tournant 14 à présent à 1500 tours/minute,
- une troisième étape "ET3" au cours de laquelle on incorpore les extraits 24 de Curcumine, les extraits 26 d'écorce de Saule Blanc, les extraits 28 de racine de Yucca, les extraits 30 de Boswellia, l'acide citrique 32 et le Sorbate 34 de potassium et au cours de laquelle on soumet le mélange à l'action du mélangeur rotatif 14 tournant de nouveau à 500 tours/minute,

- une quatrième étape "ET4" au cours de laquelle on incorpore la gomme de Xanthane 36 et au cours de laquelle on soumet le mélange à l'action du mélangeur rotatif 14 tournant à nouveau à 1500 tours/minute,
- une cinquième étape (non représentée) de refroidissement au cours de laquelle on laisse refroidir le mélange 18 jusqu'à ce que sa température atteigne la température ambiante.

On remarquera que dans le mode de réalisation préféré de l'invention, l'ensemble des quatre premières étapes "ET1 ", "ET2", "ET3" et "ET4" s'étend sur une durée comprise entre 20 et 30 minutes, ceci afin de garantir une bonne homogénéisation du mélange.

L'invention propose donc un complément alimentaire particulièrement efficace permettant notamment de soulager et soutenir les articulations sensibles et/ou vieillissantes, ce qui le rend notamment particulièrement apprécié des milieux hippiques.

## Revendications

1. Complément alimentaire (18) de santé animale ou humaine à base aqueuse comportant des principes actifs d'origine végétale, **caractérisé en ce que** lesdits principes actifs comportent au moins :
- des extraits de Curcumine, de genre Curcuma, de nom binominal Curcuma Longa,
- des extraits d'écorce de Saule Blanc, de genre Salix, de nom binominal Salix Alba,
- des extraits de racine de Yucca, de genre Yucca, de nom binominal Yucca Schidigera,
- des extraits de Boswellia, de genre Boswellia, de nom binominal Boswellia Serrata,
- des extraits de Spiruline, de genre Arthrospira, de non binominal Arthrospira Platensis,
pour permettre notamment le soulagement et le soutien des articulations sensibles et/ou vieillissantes,

2. Complément alimentaire selon la revendication précédente **caractérisé en ce qu'**il est constitué d'un mélange dont les pourcentages massiques comportent 60,4% d'eau et au moins :
- 2,6% d'extraits de Curcumine,
- 1,8% d'extraits d'écorce de Saule Blanc,
- 1,8% d'extraits de racine de Yucca,
- 0.7% d'extraits de Boswellia,
- 2% d'extraits de Spiruline,
chacun de ces dosages étant réalisé avec une marge d'erreur de 0.1 % en excès ou en défaut.

3. Complément alimentaire selon la revendication précédente, **caractérisé en ce que** :
- les extraits de Curcumine comportent une teneur de 93 à 97% de Curcumine,
- les extraits d'écorce de Saule Blanc sont constitués de plus de 92% d'extrait sec d'écorce de Saule Blanc.

4. Complément alimentaire selon l'une des revendications précédentes, **caractérisé en ce qu'**il comporte des principes inactifs, notamment des agents de goût et/ou acidifiants qui comportent au moins :
- de l'acide citrique,
- des arômes de fruit,
- du Sorbate de potassium
- de la Gomme de Xanthane
- du Saccharose

5. Complément alimentaire selon la revendication précédente, **caractérisé en ce que** l'arôme de fruit est un arôme de pomme verte.

6. Complément alimentaire selon la revendication précédente prise en combinaison avec la revendication 2, **caractérisé en ce que** le mélange comporte de surcroît des pourcentages massiques de :
- 2,3% d'acide citrique,
- 2% d'arôme de pomme verte,
- 0,3% de Sorbate de potassium
- 1% de Gomme de Xanthane
- 25,1% de Saccharose
chacun de ces dosages étant réalisé avec une marge d'erreur de 0.1 % en excès ou en défaut.

7. Procédé de fabrication d'un complément alimentaire équin à base aqueuse comportant au moins :
- des extraits de Curcuma, de genre Curcuma, de nom binominal Curcuma Longa,
- des extraits d'écorce de Saule Blanc, de genre Curcuma, de nom binominal Salix Alba,
- des extraits de racine de Yucca, de genre Yucca, de nom binominal Yucca Schidigera,
- des extraits de Boswellia, de genre Boswellia, de nom binominal Boswellia Serrata,
- de l'acide citrique,
- des arômes de pomme verte,
- du Sorbate de potassium,
- de la Gomme de Xanthane,
- du Saccharose,
**caractérisé en ce qu'**il comporte successivement :
- une première étape (ET1) de préparation de la base aqueuse au cours de laquelle on chauffe l'eau à 40°C et au cours de laquelle on la soumet à l'action d'un mélanger (14) rotatif tournant à 500 tours/minute,
- une deuxième étape (ET2) d'incorporation au cours de laquelle on incorpore les arômes (20) de pomme verte et le Saccharose (22) et au cours de laquelle on soumet le mélange à l'action du mélangeur rotatif (14) tournant à 1500 tours/minute,
- une troisième étape (ET3) au cours de laquelle on incorpore les extraits (24) de Curcumine, les extraits (26) d'écorce de Saule Blanc, les extraits (28) de racine de Yucca, les extraits (30) de Boswellia, l'acide citrique (32) et le Sorbate de potassium (34) et au cours de laquelle on soumet le mélangé à l'action du mélangeur rotatif (14) tournant à 500 tours/minute,
- une quatrième étape (ET4) au cours de laquelle on incorpore la gomme de Xanthane (36) et au cours de laquelle on soumet le mélange à l'action du mélangeur rotatif (14) tournant à 1500 tours/minute,
- une cinquième étape de refroidissement au cours de laquelle on laisse refroidir le mélange jusqu'à ce que sa température atteigne la température ambiante.

8. Procédé selon la revendication précédente, **caractérisé en ce que** l'ensemble des quatre premières étapes (ET1, ET2, ET3, ET4) s'étend sur une durée comprise entre 20 et 30 minutes.

9. Procédé selon la revendication précédente, **caractérisé en ce qu'**au cours des quatre premières étapes (ET1, ET2, ET3, ET4), on utilise un mélangeur rotatif pourvu d'une hélice quadripale.

## Patentansprüche

1. Nahrungsergänzungsmittel (18) für die Gesundheit von Tieren oder Menschen, wobei dieses auf wässriger Grundlage ist und Wirkstoffe pflanzlicher Herkunft aufweist, **dadurch gekennzeichnet, dass** die Wirkstoffe mindestens Folgendes aufweisen:
- Curcuminextrakte, von der Gattung Curcuma, mit dem wissenschaftlichen Namen Curcuma Longa,
- Extrakte der Silberweidenrinde, von der Gattung Salix, mit dem wissenschaftlichen Namen Salix Alba,
- Extrakte der Yuccawurzel, von der Gattung Yucca, mit dem wissenschaftlichen Namen Yucca Schidigera,
- Boswellia-Extrakte, von der Gattung Boswellia, mit dem wissenschaftlichen Namen Boswellia Serrata,
- Spirulinextrakte, von der Gattung Arthrospira, mit dem wissenschaftlichen Namen Arthrospira Platensis,
wobei dieses insbesondere lindernd und unterstützend auf empfindliche und/oder alternde Gelenke wirken kann.

2. Nahrungsergänzungsmittel nach dem vorhergehenden Anspruch, **dadurch gekennzeichnet, dass** es aus einer Mischung besteht, deren Prozentanteile nach Masse 60,4 % an Wasser aufweisen sowie mindestens
- 2,6 % an Curcuminextrakten,
- 1,8 % an Extrakten Silberweidenrinde,
- 1,8 % an Extrakten der Yuccawurzel,
- 0,7 % an Boswellia-Extrakten,
- 2 % an Spirulinextrakten,
wobei die Bestimmungen jeweils mit einer Fehlerspanne von 0,1 % nach oben oder nach unten durchgeführt werden.

3. Nahrungsergänzungsmittel nach dem vorhergehenden Anspruch, **dadurch gekennzeichnet, dass**:
- die Curcuminextrakte einen Gehalt an Curcumin von 93 bis 97 % aufweisen,
- die Extrakte der Silberweidenrinde zu mehr als 92 % des Trockenextrakts aus Silberweidenrinde bestehen.

4. Nahrungsergänzungsmittel nach einem der vorhergehenden Ansprüche, **dadurch gekennzeichnet, dass** es Stoffe aufweist, die nicht zur Wirkung beitragen, insbesondere Geschmacksstoffe und/oder Säuerungsmittel, die mindestens Folgendes aufweisen:
- Citronensäure,
- Fruchtaromen,
- Kaliumsorbat
- Xanthangummi
- Saccharose

5. Nahrungsergänzungsmittel nach dem vorhergehenden Anspruch, **dadurch gekennzeichnet, dass** es sich bei dem Fruchtaroma um ein Aroma des Typs Grüner Apfel handelt.

6. Nahrungsergänzungsmittel nach dem vorhergehenden Anspruch in Verbindung mit dem Anspruch 2, **dadurch gekennzeichnet, dass** die Mischung darüber hinaus die folgenden Prozentanteile nach Masse aufweist:
- 2,3 % an Citronensäure,
- 2 % an Aroma des Typs Grüner Apfel,
- 0,3 % an Kaliumsorbat
- 1 % an Xanthangummi
- 25,1 % an Saccharose
wobei die Bestimmungen jeweils mit einer Fehlerspanne von 0,1 % nach oben oder nach unten durchgeführt werden.

7. Verfahren zur Herstellung eines Nahrungsergänzungsmittel für Pferde, wobei dieses auf wässriger Grundlage ist und mindestens Folgendes aufweist:
- Curcuminextrakte, von der Gattung Curcuma, mit dem wissenschaftlichen Namen Curcuma Longa,
- Extrakte der Silberweidenrinde, von der Gattung Salix, mit dem wissenschaftlichen Namen Salix Alba,
- Extrakte der Yuccawurzel, von der Gattung Yucca, mit dem wissenschaftlichen Namen Yucca Schidigera,
- Boswellia-Extrakte, von der Gattung Boswellia, mit dem wissenschaftlichen Namen Boswellia Serrata,
- Citronensäure
- Aromen des Typs Grüner Apfel,
- Kaliumsorbat,
- Xanthangummi,
- Saccharose,
**dadurch gekennzeichnet, dass** es nacheinander Folgendes aufweist:
- einen ersten Schritt (ET1) des Zubereitens der wässrigen Grundlage, im Laufe dessen das Wasser auf 40 °C erwärmt wird und im Laufe dessen sie der Einwirkung eines rotierenden Mischgeräts (14) ausgesetzt wird, dessen Drehzahl 500 Umdrehungen/Minute beträgt,
- einen zweiten Schritt (ET2) des Hinzufügens, im Laufe dessen die Aromen (20) des Typs Grüner Apfel und die Saccharose (22) hinzugefügt werden und im Laufe dessen die Mischung der Einwirkung des rotierenden Mischgeräts (14) ausgesetzt wird, dessen Drehzahl 1.500 Umdrehungen/Minute beträgt,
- einen dritten Schritt (ET3) im Laufe dessen die Curcuminextrakte (24), die Extrakte (26) der Silberweidenrinde, die Extrakte (28) der Yuccawurzel, die Boswellia-Extrakte (30), die Citronensäure (32) und das Kaliumsorbat (34) hinzugefügt werden und im Laufe dessen die Mischung der Einwirkung des rotierenden Mischgeräts (14) ausgesetzt wird, dessen Drehzahl 500 Umdrehungen/Minute beträgt,
- einen vierten Schritt (ET4), im Laufe dessen das Xanthangummi (36) hinzugefügt wird und im Laufe dessen die Mischung der Einwirkung des rotierenden Mischgeräts (14) ausgesetzt wird, dessen Drehzahl 1.500 Umdrehungen/Minute beträgt,
- einen fünften Schritt des Abkühlens, im Laufe dessen die Mischung abkühlen gelassen wird, bis ihre Temperatur die Raumtemperatur erreicht.

8. Verfahren nach dem vorhergehenden Anspruch, **dadurch gekennzeichnet, dass** die ersten vier ersten Schritte (ET1, ET2, ET3, ET4) sich insgesamt über einen Zeitraum erstrecken, der im Bereich von 20 bis 30 Minuten liegt.

9. Verfahren nach dem vorhergehenden Anspruch, **dadurch gekennzeichnet, dass** im Laufe der ersten vier Schritte (ET1, ET2, ET3, ET4) ein rotierendes Mischgerät verwendet wird, welches mit einer vierflügeligen Schraube versehen ist.

## Claims

1. Aqueous animal or human health food supplement (18) comprising plant-based active principles, **characterised in that** said active principles comprise at least:
- curcumin extracts of the genus Curcuma, having the binominal name Curcuma longa;
- white willow bark extracts of the genus Salix, having the binominal name Salix alba;
- yucca root extracts of the genus Yucca, having the binominal name Yucca schidigera;
- Boswellia extracts of the genus Boswellia, having the binominal name Boswellia serrata; and
- spirulina extracts of the genus Arthrospira having the binominal name Arthrospira platensis,
in order to enable in particular the relief and support for sensitive and/or ageing joints.

2. Food supplement according to the preceding claim, **characterised in that** it consists of a mixture, whose weight percentages comprise 60.4% water and at least:
- 2.6% of curcumin extracts,
- 1.8% of white willow bark extracts,
- 1.8% of yucca root extracts,
- 0.7% of Boswellia extracts,
- 2% of spirulina extracts,
each of these dosages being realised within a margin of error of plus or minus 0.1%.

3. Food supplement according to the preceding claim, **characterised in that**:
- the curcumin extracts comprise a content of 93 to 97% of curcumin,
- the white willow bark extracts consist of more than 92% of dry extract of white willow bark.

4. Food supplement according to one of the preceding claims, **characterised in that** it comprises inactive principals, in particular flavouring agents and/or acidifiers, which comprise at least:
- citric acid,
- fruit aromas,
- potassium sorbate
- Xanthan gum
- saccharose

5. Food supplement according to the preceding claim, **characterised in that** the fruit aroma is a green apple aroma.

6. Food supplement according to the preceding claim combined with claim 2, **characterised in that** the mixture moreover comprises the percentages by weight of:
- 2.3% citric acid,
- 2% of green apple aroma,
- 0.3% potassium sorbate
- 1% Xanthan gum
- 25.1% saccharose
each of these dosages being realised within a margin of error of plus or minus 0.1%.

7. Process for manufacturing an equine aqueous food supplement comprising at least:
- curcumin extracts of the genus Curcuma having the binominal name Curcuma longa;
- white willow bark extracts of the genus Salix, having the binominal name Salix alba;
- yucca root extracts of the genus Yucca, having the binominal name Yucca schidigera;
- Boswellia extracts of the genus Boswellia, having the binominal name Boswellia serrata,
- citric acid,
- green apple aromas,
- potassium sorbate,
- Xanthan gum,
- saccharose,
**characterised in that** said process successively comprises:
- a first step (ET1) to prepare the aqueous base in which the water is heated to 40 °C with stirring by means of a rotary mixer (14) turning at 500 rpm,
- a second step (ET2) to incorporate the green apple aromas (20) and the saccharose (22) with stirring by means of a rotary mixer (14) turning at 1500 rpm,
- a third step (ET3) to incorporate the curcumin extracts (24), the white willow bark extracts (26), the yucca root extracts (28), the Boswellia extracts (30), the citric acid (32) and the potassium sorbate (34) with stirring by means of a rotary mixer (14) turning at 500 rpm,
- a fourth step (ET4) to incorporate the Xanthan gum (36) with stirring by means of a rotary mixer (14) turning at 1500 rpm,
- a fifth step for cooling in which the mixture is left to cool down to ambient temperature.

8. Process according to the preceding claim, **characterised in that** the group of the first four steps (ET1, ET2, ET3, ET4) together extends over a period comprised between 20 and 30 minutes.

9. Process according to the preceding claim, **characterised in that** a rotary mixer equipped with a four-bladed propeller is used in the first four steps (ET1, ET2, ET3, ET4).
